# EUROPEAN PATENT SPECIFICATION

(11) **EP 3 962 501 B1**
(45) Date of publication and mention of the grant of the patent: **19.03.2025**
(21) Application number: 20798130.9
(22) Date of filing: 29.04.2020
(51) Int. Cl.: A61K 35/28, A61K 35/35, C12N 5/0775, A61P 3/08, A61P 3/10, C12N 5/071

(54) **NON-NATURALLY OCCURING THREE-DIMENSIONAL (3D) BROWN ADIPOSE-DERIVED STEM CELL AGGREGATES, AND METHODS OF GENERATING AND USING THE SAME**
NICHTNATÜRLICH VORKOMMENDE DREIDIMENSIONALE (3D), AUS BRAUNEM FETTGEWEBE GEWONNENE STAMMZELLAGGREGATE UND VERFAHREN ZUR BILDUNG UND VERWENDUNG DAVON
AGRÉGATS DE CELLULES SOUCHES DÉRIVÉES DE TISSUS ADIPEUX BRUNS TRIDIMENSIONNELS (3D) D'ORIGINE NON NATURELLE ET LEURS PROCÉDÉS DE PRODUCTION ET LEURS MÉTHODES D'UTILISATION

(30) Priority: 29.04.2019 US 201962840096 P
(43) Date of publication of application: 09.03.2022
(73) Proprietor: Biorestorative Therapies, Inc., Melville, NY 11747 (US)
(72) Inventor: SILVA, Francisco Javier, Halesite, NY 11743 (US)
(74) Representative: Reichert & Lindner Partnerschaft Patentanwälte
(86) International application number: PCT/US2020/030520
(87) International publication number: WO 2020/223381

(56) References cited:
- US-A1- 2012 321 671
- US-A1- 2013 209 418
- US-A1- 2015 202 234
- US-A1- 2017 191 035
- FRANCISCO J SILVA ET AL: "Metabolically Active Human Brown Adipose Tissue Derived Stem Cells", STEM CELLS, vol. 32, no. 2, 13 February 2014 (2014-02-13), pages 572 - 581, XP055127776, ISSN: 1066-5099, DOI: 10.1002/stem.1595
- ANDREA M. UNSER ET AL: "Opportunities and challenges in three-dimensional brown adipogenesis of stem cells", BIOTECHNOLOGY ADVANCES., vol. 33, no. 6, 1 November 2015 (2015-11-01), GB, pages 962 - 979, XP055300324, ISSN: 0734-9750, DOI: 10.1016/j.biotechadv.2015.07.005

## Description

### FIELD

The present application provides methods of making non-naturally occurring 3D brown adipose-derived stem cell (BADSC) aggregates consisting of brown adipose derived stem cells that express one or more brown adipocyte gene in the absence of differentiation medium.

### BACKGROUND

The prevalence of metabolic disorders, such as obesity, has increased dramatically over the past several decades and has become a pandemic. By 2030, more than 50% of Americans will suffer from obesity, resulting in over a 500 billion dollar loss in economic productivity. Obesity is a major risk factor for type II diabetes mellitus, hypertension, cardiovascular disease, osteoarthritis, and certain forms of cancer. Current therapeutic approaches, such as caloric restriction and exercise, which rely mainly on patient's will power to reduce energy intake and/or increase energy expenditure, are of limited effectiveness in obese patients. Bariatric surgery is the only clinically proven therapy in terms of weight loss and decreased morbidity/mortality in patients with a body mass index (BMI) over 40; however, it has associated risks, high costs, and requires proper management of patient's nutrition and physical activity. Despite efforts from researchers and medical professionals worldwide who have been trying to address obesity and other metabolic disorders, there is still a need for alternative ways to increase energy expenditure that could augment the current therapeutic options for treating obese patients and patients with other metabolic disorders.

FRANCISCO J SILVA ET AL: "Metabolically Active Human Brown Adipose Tissue Derived Stern Cells", STEM CELLS, vol. 32, no. 2, 13 February 2014 (2014-02-13), pages 572-581, XP055127776, ISSN: 1066-5099, DOI: 10.1002/stem.1595, can be seen as closest prior art document and discloses differentiating brown adipose stem cells in a differentiation medium including dexamethasone, isobutylmethylxanthine, rosiglitazone, and T3. The BADSC and a scaffold are required for three-dimensional formation.
ANDREA M. UNSER ET AL: "Opportunities and challenges in threedimensional brown adipogenesis of stem cells", BIOTECHNOLOGYADVANCES-, vol. 33, no. 6, 1 November 2015 (2015-11-01), pages 962-979, XP055300324, GB, ISSN: 0734-9750, DOI: 10.1016/j.biotechadv.2015.07.005, can also be seen as closest prior art document and discloses differentiating brown adipose stem cells in an adipogenic cocktail including dexamethasone and isobutylmethylxanthine. The BADSC and a scaffold are required for a three-dimensional formation (or hydrogel, or alginate microsphere).
US 2017/191035 A1 also discloses differentiating brown adipose stem cells in a differentiation medium (differentiation factor cocktail) and the production of cell aggregates composed of adipose stem cells (ASC) and endothelial cells.
US 2013/209418 A1 discloses no non-naturally occurring three-dimensional brown adipose derived stem cell aggregates, but differentiating brown adipose stem cells in an adipogenic induction medium including dexamethasone, isobutylmethylxanthine, rosiglitazone, and T3.
US 2012/321671 A1 serves a different object, namely modulating heat loss in a neonatal human subject and discloses differentiating brown adipose stem cells in a differentiation medium including dexamethasone, isobutylmethylxanthine, and rosiglitazone. The natural BADS are cultured as a three dimensional sheets employing a 'self-assembly' culture methodology and biocompatible, preferably micro-sized, carriers.
US 2015/0202234 A1 discloses cell aggregates consisting of adipose stem cells and endothelial cells. During the aggregation process, the endothelial cells migrate to the middle of the aggregate while the adipose stem cells remain on the outside. This particular 3D conformation is important because it promotes collagen production, close cell-cell association, and rounded cell shape, which in turn promotes vascularization of the endothelial cells and differentiation of the adipose stem cells to brown fat. The aggregates comprise BADSC composed of adipose stem cells (ASC) and, necessarily, endothelial cells. The importance of using endothelial cells when producing the aggregates is emphasized.

### SUMMARY

This section provides a general summary of the disclosure and is not comprehensive of its full scope or all of its features. The invention is set out in the appended set of claims.

Provided herein is a non-naturally occurring three-dimensional brown adipose derived stem cell aggregate, which is not part of the invention. The three-dimensional brown adipose derived stem cell aggregate comprises brown adipose-derived stem cells that express one or more brown adipocyte genes in the absence of differentiation medium.

Also provided herein is an encapsulation system, which is not part of the invention, comprising a non-naturally occurring three-dimensional brown adipose derived stem cell aggregate. The three-dimensional brown adipose derived stem cell aggregate comprises brown adipose-derived stem cells that express one or more brown adipocyte gene in the absence of differentiation medium.

Also provided herein is a method of making a non-naturally occurring three-dimensional brown adipose derived stem cell aggregate according to claim 1. The method comprises: loading brown adipose derived stem cells grown in a two-dimensional (2D) culture into a non-adherent culture plate, and centrifuging the non-adherent culture plate to uniformly position the brown adipose- derived stem cells in the non-adherent culture plate, thereby forming three-dimensional brown adipose derived stem cell aggregates, wherein the aggregates consist of brown adipose derived stem cells.

Also provided herein is a method of making a three-dimensional brown adipose tissue in an encapsulation system. The method comprises: forming non-naturally occurring three-dimensional brown adipose derived stem cell aggregates, loading the non-naturally occurring three-dimensional brown adipose derived stem cell aggregates into the encapsulation system, differentiating the non-naturally occurring three-dimensional brown adipose derived stem cell aggregates into brown adipose tissue in a first differentiation medium, and differentiating the non-naturally occurring three-dimensional brown adipose derived stem cell aggregates into brown adipose tissue in a second differentiation medium.

Also provided herein is a method of treating a patient with a disorder. The method comprises: forming non-naturally occurring three-dimensional brown adipose derived stem cell aggregates; loading the non-naturally occurring three-dimensional brown adipose derived stem cell aggregates into an encapsulation system; differentiating the non-naturally occurring three- dimensional brown adipose derived stem cell aggregates into brown adipose tissue in a first differentiation medium; differentiating the non-naturally occurring three-dimensional brown adipose derived stem cell aggregates into brown adipose tissue in a second differentiation medium; and delivering the brown adipose tissue to the patient with the disorder.

The foregoing summary is illustrative only and is not intended to be in any way limiting. In addition to the illustrative examples and features described herein, further aspects, examples, objects and features of the disclosure will become fully apparent from the drawings and the detailed description and the claims.

### BRIEF DESCRIPTION OF THE DRAWINGS

The patent or patent application contains at least one drawing executed in color. Copies of this patent or patent application publication with color drawing(s) will be provided by the Office upon request and payment of the necessary fee.

Various aspects of non-naturally occurring 3D brown adipose-derived stem cell (BADSC) aggregates, methods of making the 3D BADSC aggregates, and methods of using the 3D BADSC aggregates are disclosed and described in this specification and can be better understood by reference to the accompanying figures, in which:

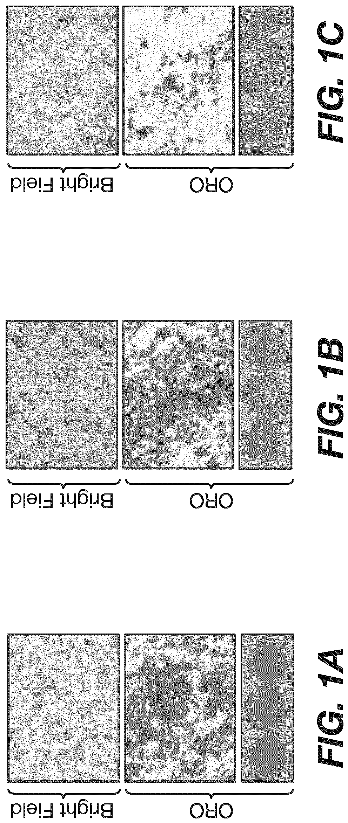

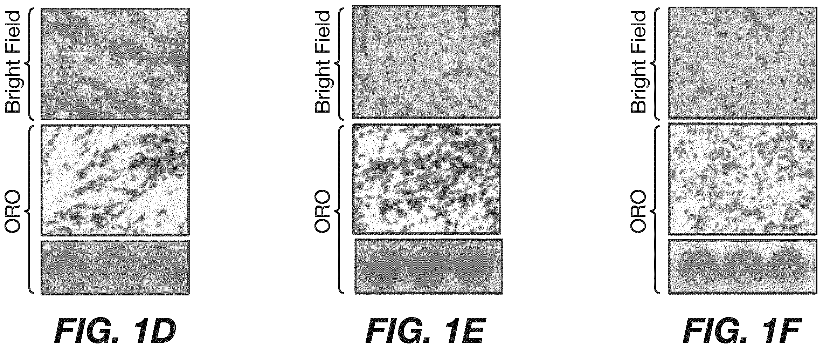

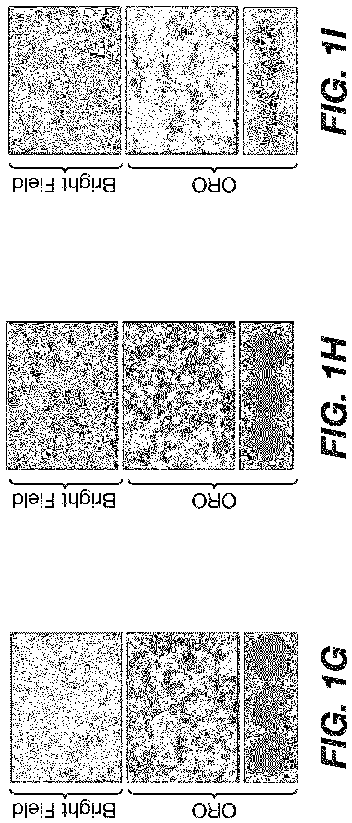

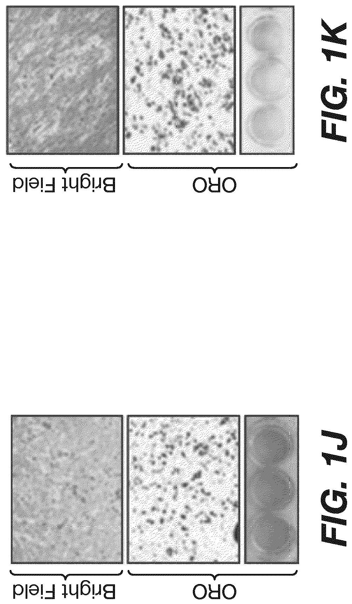

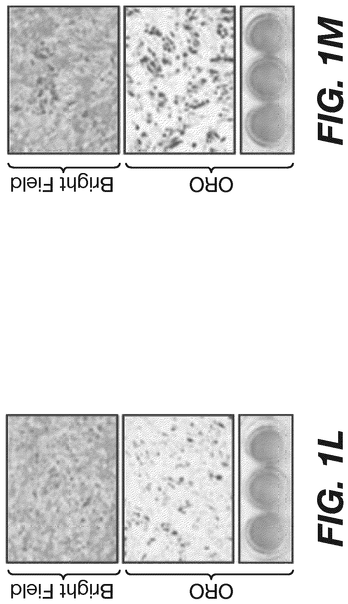

Figures 1A to 1M show thirteen human brown adipose-derived mesenchymal stem cell (BADSC) populations (BF-1 to BF-13) isolated from subcutaneous supraclavicular and mediastinal adipose tissue biopsies and evaluated for their ability to differentiate into brown adipocytes. These BADSC populations were evaluated via bright field (top panels) and oil red O staining (ORO) (middle panels).
Figure 1A shows human BADSC population BF-1.
Figure 1B shows human BADSC population BF-2.
Figure 1C shows human BADSC population BF-3.
Figure 1D shows human BADSC population BF-4.
Figure 1E shows human BADSC population BF-5.
Figure 1F shows human BADSC population BF-6.
Figure 1G shows human BADSC population BF-7.
Figure 1H shows human BADSC population BF-8.
Figure 1I shows human BADSC population BF-9.
Figure 1J shows human BADSC population BF-10.
Figure 1K shows human BADSC population BF-11.
Figure 1L shows human BADSC population BF-12.
Figure 1M shows human BADSC population BF-13.
Figure 2A shows UCP-1 expression for BADSC population BF-1 before differentiation in AD-1 culture medium (Pre Diff AD-1); BADSC population BF-1 post differentiation in AD-1 culture medium (Post Diff AD-1); BADSC population BF-1 before differentiation in AD-2 culture medium (Pre Diff AD-2); and BADSC population BF-1 after differentiation in AD-2 culture medium (Post Diff AD-2). Human brown adipose tissue was used as a positive control (Human BAT). Human white adipose tissue was used as a negative control (SubQ WAT and Visc. WAT).
Figure 2B shows the expression of UCP1 mRNA via qPCR for the BADSC population BF-1, 15 days after differentiation in either StemPro^{™}, AD-1, or AD-2 culture medium.
Figure 2C shows the expression of FABP4 mRNA via qPCR for the BADSC population BF-1, 15 days after differentiation in either StemPro^{™}, AD-1, or AD-2 culture medium.
Figure 2D shows the expression of ADIPSIN mRNA via qPCR for the BADSC population BF-1, 15 days after differentiation in either StemPro^{™}, AD-1, or AD-2 culture medium.
Figure 2E shows the expression of LEPTIN mRNA via qPCR for the BADSC population BF-1, 15 days after differentiation in either StemPro^{™}, AD-1, or AD-2 culture medium.
Figure 2F shows the BADSC population BF-1 differentiated in AD-2 culture medium. Fifteen days after differentiation induction, cells were fixed and immunostained for Perilipin (green) using an antibody that binds Perilipin.
Figure 2G shows the BADSC population BF-1 differentiated in AD-2 culture medium. Fifteen days after differentiation induction, cells were fixed and immunostained for Perilipin (green) using an IgG control antibody.
Figure 2H shows the BADSC population BF-1 differentiated in AD-2 culture medium. Fifteen days after differentiation induction, cells were fixed and immunostained for UCP1 (red) using an antibody that binds UCP1.
Figure 2I shows the BADSC population BF-1 differentiated in AD-2 culture medium. Fifteen days after differentiation induction, cells were fixed and immunostained for UCP1 (red) using an IgG control antibody.
Figure 2J shows the BADSC population BF-1 differentiated in AD-2 culture medium. Fifteen days after differentiation induction, cells were fixed and counterstained with DAPI (blue).
Figure 2K shows the BADSC population BF-1 differentiated in AD-2 culture medium. Fifteen days after differentiation induction, cells were fixed and counterstained with DAPI (blue).
Figure 2L shows the BADSC population BF-1 differentiated in AD-2 culture medium. Fifteen days after differentiation induction, cells were fixed and immunostained for Mitochondria (green) using an antibody that binds Mitochondria.
Figure 2M shows the BADSC population BF-1 differentiated in AD-2 culture medium. Fifteen days after differentiation induction, cells were fixed and immunostained for Mitochondria (green) using an IgG control antibody.
Figure 2N shows the BADSC population BF-1 differentiated in AD-2 culture medium. Fifteen days after differentiation induction, cells were fixed and immunostained for UCP1 (red) using an antibody that binds UCP1.
Figure 2O shows the BADSC population BF-1 differentiated in AD-2 culture medium. Fifteen days after differentiation induction, cells were fixed and immunostained for UCP1 (red) using an IgG control antibody.
Figure 2P shows the BADSC population BF-1 differentiated in AD-2 culture medium. Fifteen days after differentiation induction, cells were fixed and counterstained with DAPI (blue).
Figure 2Q shows the BADSC population BF-1 differentiated in AD-2 culture medium. Fifteen days after differentiation induction, cells were fixed and counterstained with DAPI (blue).
Figure 2R shows the quantification of adipocyte differentiation efficiency (% differentiation) for the BADSC population BF-1 defined as the percentage of perilipin positive cells using DAPI to quantify the total number of cells per field of view. Figure 2R also shows the quantification of brown adipocyte differentiation efficiency (% Brown) for the BADSC population BF-1 defined as the percentage of perilipin positive cells that are positive for UCP1.
Figure 3A shows gene expression levels for an adipocyte marker (PPARa) determined by qPCR for (1) BADSC population BF-1 in 2D, (2) BADSC population BF-1 in 3D at 24 hours; and (3) BADSC population BF-1 in 3D at 48 hours.
Figure 3B shows gene expression levels for an adipocyte marker (PPARg) determined by qPCR for (1) BADSC population BF-1 in 2D, (2) BADSC population BF-1 in 3D at 24 hours; and (3) BADSC population BF-1 in 3D at 48 hours.
Figure 3C shows gene expression levels for a brown adipocyte marker (PGC1a) determined by qPCR for (1) BADSC population BF-1 in 2D, (2) BADSC population BF-1 in 3D at 24 hours; and (3) BADSC population BF-1 in 3D at 48 hours.
Figure 3D shows gene expression levels for an adipocyte marker (PGC1b) determined by qPCR for (1) BADSC population BF-1 in 2D, (2) BADSC population BF-1 in 3D at 24 hours; and (3) BADSC population BF-1 in 3D at 48 hours.
Figure 3E shows gene expression levels for an adipocyte marker (PRDM16) determined by qPCR for (1) BADSC population BF-1 in 2D, (2) BADSC population BF-1 in 3D at 24 hours; and (3) BADSC population BF-1 in 3D at 48 hours.
Figure 3F shows gene expression levels for an adipocyte marker (CEBPd) determined by qPCR for (1) BADSC population BF-1 in 2D, (2) BADSC population BF-1 in 3D at 24 hours; and (3) BADSC population BF-1 in 3D at 48 hours.
Figure 3G shows gene expression levels for an adipocyte marker (CEBPb) determined by qPCR for (1) BADSC population BF-1 in 2D, (2) BADSC population BF-1 in 3D at 24 hours; and (3) BADSC population BF-1 in 3D at 48 hours.
Figure 3H shows gene expression levels for an adipocyte marker (CEBPa) determined by qPCR for (1) BADSC population BF-1 in 2D, (2) BADSC population BF-1 in 3D at 24 hours; and (3) BADSC population BF-1 in 3D at 48 hours.
Figure 3I shows gene expression levels for an adipocyte marker (TFAM) determined by qPCR for (1) BADSC population BF-1 in 2D, (2) BADSC population BF-1 in 3D at 24 hours; and (3) BADSC population BF-1 in 3D at 48 hours.
Figure 4A shows a schematic diagram of the three-step 3D brown adipocyte differentiation protocol in an encapsulation system.
Figure 4B shows a micrograph (5X magnification) of BAGs, 24 hours post formation.
Figure 4C shows a micrograph (5X magnification) of BAGs, after collection.
Figure 4D shows a photograph of the encapsulation medical device, Encaptra^{®} EN20.
Figure 4E shows a photograph of the encapsulation medical device, Encaptra^{®} EN20, loaded with BAGs.
Figure 4F shows a micrograph (10X magnification) of live BAGs differentiating inside the encapsulation medical device, Encaptra^{®} EN20.
Figure 4G shows cross sections of BAGs differentiating inside the encapsulation medical device, Encaptra^{®} EN20. The cross sections were shown using hematoxylin and eosin staining.
Figure 4H shows cross sections of BAGs differentiating inside the encapsulation medical device, Encaptra^{®} EN20. The cross sections were shown using bright field.
Figure 4I shows cross sections of BAGs differentiating inside the encapsulation medical device, Encaptra^{®} EN20. The cross sections were shown using immunostaining for Perilipin (green), UCP1 (red) and counterstaining with DAPI (blue).
Figure 4J shows cross sections of BAGs differentiating inside the encapsulation medical device, Encaptra^{®} EN20. The cross sections were shown using staining with DAPI (blue).
Figure 4K shows cross sections of BAGs differentiating inside the encapsulation medical device, Encaptra^{®} EN20. The cross sections were shown using immunostaining for Perilipin (green).
Figure 4L shows cross sections of BAGs differentiating inside the encapsulation medical device, Encaptra^{®} EN20. The cross sections were shown using immunostaining for UCP1 (red).
Figure 4M shows gene expression levels for an adipocyte marker (FABP4) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4N shows gene expression levels for an adipocyte marker (ADIPSIN) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4O shows gene expression levels for an adipocyte marker (PPARg) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4P shows gene expression levels for an adipocyte marker (CEBPa) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4Q shows gene expression levels for an adipocyte marker (LEPTIN) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4R shows gene expression levels for a brown adipocyte marker (UCP1) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4S shows gene expression levels for a brown adipocyte marker (PGC1a) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4T shows gene expression levels for a brown adipocyte marker (ELOVL3) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4U shows gene expression levels for a brown adipocyte marker (CIDEA) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 4V shows gene expression levels for a brown adipocyte markers (COX10) determined by qPCR. RNA that was used for the qPCR was collected from BAGs at D0 (undifferentiated) and from the BAGs located within the encapsulation medical device, Encaptra^{®} EN20, at D25 (see Figure 4A).
Figure 5A is a graph showing Glucose Tolerance Testing (GTT) of mice transplanted with BAT encapsulated in matrigel compared to mice transplanted with only matrigel.
Figures 5B-C are tables representing the data generated in the GTT experiment described in Figure 5A.

### DETAILED DESCRIPTION

Certain exemplary aspects of the present disclosure will now be described to provide an overall understanding of the principles of the structure, function, manufacture, and use of the non-naturally occurring three-dimensional brown adipose-derived stem cell aggregates and methods disclosed herein. One or more examples of these aspects are illustrated in the accompanying drawings. Those of ordinary skill in the art will understand that the non-naturally occurring three-dimensional brown adipose-derived stem cell aggregates and methods specifically described herein and illustrated in the accompanying drawings are non-limiting exemplary aspects and that the scope of the various examples of the present disclosure is defined solely by the claims. The features illustrated or described in connection with one exemplary aspect may be combined with the features of other aspects. Such modifications and variations are intended to be included within the scope of the present disclosure.

### Non-naturally occurring three-dimensional brown adipose derived stem cell aggregates and methods of making the non-naturally occurring 3D BAGs

Non-naturally occurring three-dimensional BADSC aggregates (not part of the invention) or "BAGs" are disclosed herein. The BAGs are 3D structures formed from BADSC after the BADSC are removed from their two-dimensional (2D) culture in cell adherent tissue culture flasks, added to non-adherent culture plates, and centrifuged. After centrifugation, the aggregates are uniform. Uniform cell aggregates provide a more efficient and consistent differentiation and are easier to load into encapsulation systems. Furthermore, uniform aggregation provides a more accurate cell number and a more accurate dosage.

BADSCs grown in 2D are the natural state of the BADSC whenever the cells expand in tissue adherent cell culture flasks. BADSCs cultured in growth media in 2D are multipotent and function as a stem cell. The BADSC grown in 2D cannot form aggregates because they attach to the cell culture flask and then differentiate into an unwanted non-adipose cell type and ultimately induce apoptotic cascade and cell death.

BADSC cannot form cell aggregates in 2D culture, but whenever the BADSC are removed from their 2D tissue adherent environment and placed in a non-adherent environment, the cells form 3D aggregates, as described above. The BADSC when aggregated form clusters of cells that are able to communicate with each other and their environment in 3D.

The BAGs can be expanded and further aggregated to become artificial brown adipose tissue or artificial white adipose tissue. The BAGs can become white adipose tissue if differentiated in AD-1. AD-1 is a serum based differentiation medium composed of DMEM low
glucose (Gibco, Thermo Fisher Scientific) supplemented with 10% fetal bovine serum (FBS, HyClone, GE Healthcare, Life Sciences, Little Chalfont, Buckinghamshire, UK), 5 µM dexamethasone (MP Biomedicals, Santa Ana, California, USA), 500 µM 3-isobutyl-1-methylxanthine (IBMX, Sigma-Aldrich, St. Louis, Missouri, USA), 860 nM insulin (Gibco, Thermo Fisher Scientific), 125 nM indomethacin (Sigma-Aldrich), 1 nM triiodothyronine (T3, Sigma-Aldrich), 1 µM rosiglitazone (Sigma-Aldrich), 100 units/ml of penicillin, 100 µg/ml of streptomycin (Gibco, Thermo Fisher Scientific), and 2 mM L-glutamine (Gibco, Thermo Fisher Scientific)

The BAGs can become brown adipose tissue if differentiated in AD-2. AD-2 is a two-step xeno-free, serum free, chemically defined differentiation medium. In a first step, BADSC are grown in a first differentiation medium, AD-2 DIFF-1 culture medium, which comprises DMEM / Ham's F12 Media (1:1) (Lonza Group AG, Basel, Switzerland), 25 mM HEPES Buffer (Lonza Group AG), 2 mM L-glutamine (Gibco, Thermo Fisher Scientific), 1 µM dexamethasone (MP Biomedicals), 100 µM IBMX (Sigma-Aldrich), 860 nM insulin (Gibco, Thermo Fisher Scientific), 0.2 nM T3 (Sigma-Aldrich), 10 µg/ml apo-transferrin (Sigma-Aldrich), 100 units/ml of penicillin and 100 µg/ml of streptomycin (Gibco, Thermo Fisher Scientific). In a second step, after three days the AD-2 DIFF-1 culture medium was replaced with a second differentiation medium, AD-2 DIFF-2, a xeno-free, serum free, chemically defined differentiation medium, which comprises DMEM / Ham's F12 Media (1:1) (Lonza Group AG), 25 mM HEPES Buffer (Lonza Group AG), 2 mM L-glutamine (Gibco, Thermo Fisher Scientific), 860 nM insulin (Gibco, Thermo Fisher Scientific), 0.2 nM T3 (Sigma-Aldrich), 10 µg/ml apo-transferrin (Sigma-Aldrich), 100 units/ml of penicillin and 100 µg/ml of streptomycin (Gibco, Thermo Fisher Scientific) and 100 nM rosiglitazone.

These BAGs can serve as cell factories that can produce white or brown extracellular biologics (e.g., exosomes, microRNAs, cytokines, proteins, adipokines).

### Gene expression of 3D BAGs

BAGs upregulate adipocyte markers (PPARα, PPARγ, PGC1β, PRDM16, CEBPd, CEBPb, CEBPa, and TFAM) and brown adipocyte markers (PGC1α) in the absence of differentiation medium.

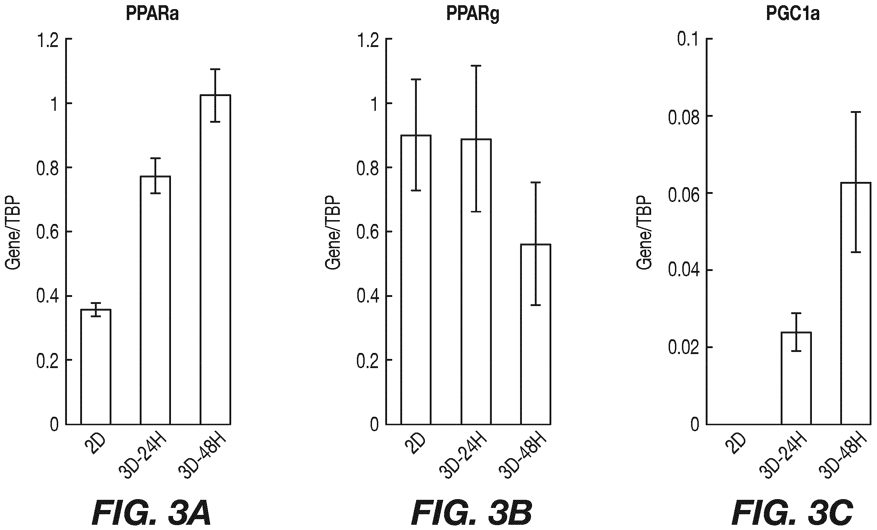

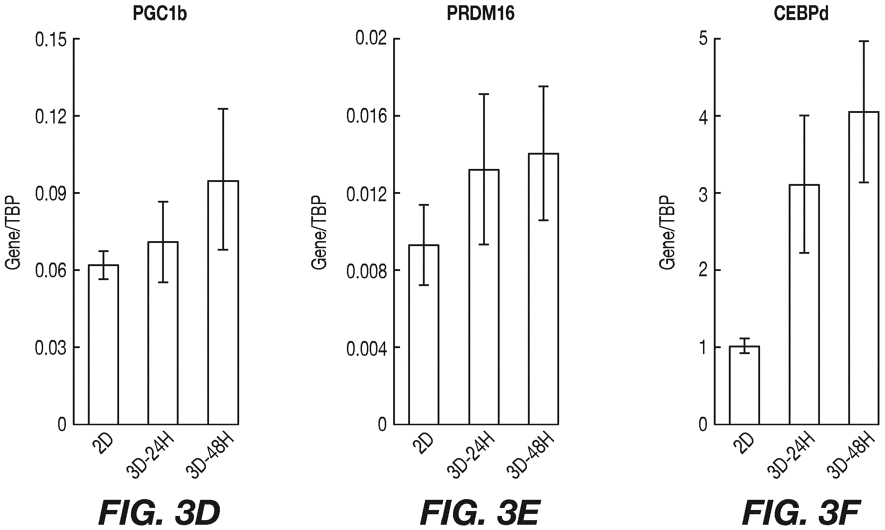

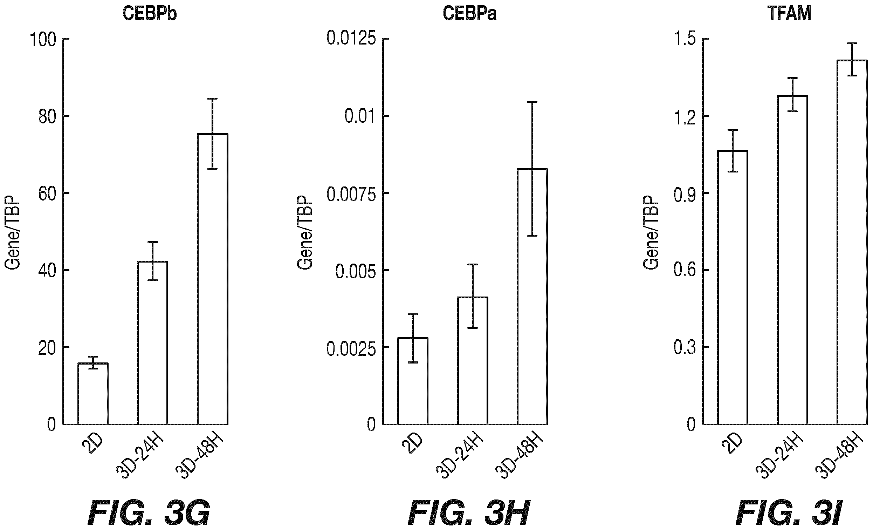

The formation of BAGs resulted in an increased expression of transcription factors and co-factors from the CEBP and PPAR families, which are master regulators of adipogenesis and browning (Figures 3A to 3I). "Browning" refers to the BAGs ability to express UCP-1 post-differentiation in AD-2 medium.

The early adipocyte differentiation transcription factors CEBPD and CEBPB were increased after 24 hours in 3D culture whereas CEBPA was significantly increased after 48 hours in 3D culture. PPARα, a master regulator of fatty acid oxidation, and PGC1α, a regulator of mitochondrial respiration and heat production in brown adipocyte, were both increased after 24 hours in 3D cultures whereas, no significant changes in the expression of PPARy, PRDM16, TFAM or PGC1β were observed.

These data suggest that the formation of 3D BAGs commits BADSC aggregates to adipogenesis and a brown adipose phenotype and so the BAGs have started down the path of brown adipose differentiation in the absence of adipocyte differentiation medium.

### Encapsulation systems as delivery systems for BAT

Transplanting brown adipose tissue (BAT) into humans, in order to increase BAT mass and/or activity, has emerged as a potential way to increase energy expenditure by energy wasting. This approach of transplanting BAT into humans can be used to treat metabolic disorders, endocrine disorders, cardiovascular disorders, and liver diseases. Therefore, a method according to claims 4 to 8 of delivering BAT for transplantation using 3D BAGs loaded into encapsulation systems was sought and is disclosed herein.

Several different encapsulation systems (not part of the invention) can be loaded with BAGs and used to deliver the BAT for transplantation such as alginate microcapsules, cellulose hydrogels, red blood cells, porous polymer membranes, 3D biological scaffolds, Afibromer^{™} polymers (Sigilon Therapeutics, Cambridge, Massachussetts, USA), PEG-based hydrogels, non-hydrogel beads, and matrigel.

The encapsulation systems described herein allow the BAGs to produce extracellular factors that can interact with the host environment, such as proteins, cytokines, microRNAs, cytokines, exosomes, and cell specific secretome.

The encapsulation systems described herein are manufactured from implantable- grade materials or biologies and are selected for long-term biocompatibility.

The encapsulation systems described herein provide a bidirectional exchange of nutrients and molecules such as glucose, fatty acids, cytokines, adipokines, and hormones.

In certain examples, the encapsulation system can be an encapsulation medical device. In other examples, the encapsulation medical device can be an FDA-approved, immune-protecting, easily retrievable encapsulation medical device, such as the Encaptra^{®} Drug Delivery System (Viacyte, San Diego, California, USA). This device is manufactured from implant-grade materials specifically selected for long-term biocompatibility and allows for bidirectional
exchange of nutrients and molecules such as glucose, fatty acids, and hormones. The encapsulation medical device provides a barrier between the host and the transplanted cells and therefore should prevent immune rejection of BAT while increasing safety and preventing transplanted cells to migrate out of the encapsulation medical device.

### Method of making 3D BAT in an encapsulation system

Disclosed herein is a method of making 3D BAT in an encapsulation system according to claims 4 to 8. An embodiment of the method comprises (1) forming non-naturally occurring three-dimensional brown adipose derived stem cell aggregates, (2) loading the non-naturally occurring three-dimensional brown adipose derived stem cell aggregates into the encapsulation system, (3) differentiating the non-naturally occurring three-dimensional brown adipose derived stem cell aggregates into brown adipose tissue in a first differentiation medium, and (4) differentiating the non-naturally occurring three- dimensional brown adipose derived stem cell aggregates into brown adipose tissue in a second differentiation medium. The "first differentiation medium" can also be referred to herein as AD- 2 DIFF-1 culture medium. The "second differentiation medium" can also be referred to herein as AD-2 DIFF-2 culture medium.

### Methods of treatment (not part of the invention)

Methods of treating patients with disorders are disclosed herein, being not part of the invention. Methods of treating patients with metabolic disorders, endocrine disorders, cardiovascular disorders, and liver diseases are disclosed herein. Examples of metabolic disorders can include, but are not limited to, diabetes and obesity. Examples of endocrine disorders can include, but are not limited to, acromegaly, Addison's Disease, adrenal cancer, adrenal disorders, anaplastic thyroid cancer, Cushing's Syndrome, De Quervain's Thyroiditis, diabetes (e.g., type 1 diabetes, type 2 diabetes, gestational diabetes, maturity onset diabetes of the young), follicular thyroid cancer, goiters, Graves' Disease, growth disorders, growth hormone deficiency, Hashimoto's Thyroiditis, heart disease, Hurthle Cell Thyroid Cancer, hyperglycemia, hyperparathyroidism, hyperthyroidism, hypoglycemia, hypoparathyroidism, hypothyroidism, low testosterone, medullary thyroid cancer, MEN 1, MEN 2A, MEN 2B, menopause, metabolic syndrome, obesity, osteoporosis, papillary thyroid cancer, parathyroid diseases, pheochromocytoma, pituitary disorders, pituitary tumors, polycystic ovary syndrome, prediabetes, reproduction, silent thyroiditis, thyroid cancer, thyroid diseases, thyroid nodules, thyroiditis, turner syndrome, insulin resistance, hypertension, central obesity, hypertriglyceridemia (e.g., high serum triglycerides), dyslipidemia, low serum HDL, lipodystrophy. Examples of cardiovascular disorders can include, but are not limited to, coronary artery disease, peripheral artery disease, carotid artery disease, peripheral artery (arterial) disease, aneurysm, atherosclerosis, renal artery disease, Raynaud's disease (Raynaud's phenomenon), Buerger's disease, peripheral venous disease, cerebrovascular disease (e.g., stroke), venous blood clots, and blood clotting disorders, cardiomyopathy, hypertensive heart disease (e.g., diseases of the heart secondary to high blood pressure or hypertension). Examples of liver disease can include, but are not limited to, simple fatty liver disease, nonalcoholic steatohepatitis (NASH), and alcohol-related fatty liver disease (ALD).

Disclosed herein is a method of treating a patient with a metabolic disorder. The method comprises: forming non-naturally occurring three-dimensional brown adipose derived stem cell aggregates; loading the non-naturally occurring three-dimensional brown adipose derived stem cell aggregates into an encapsulation system; differentiating the non-naturally occurring three-dimensional brown adipose derived stem cell aggregates into brown adipose tissue in a first differentiation medium; differentiating the non-naturally occurring three-dimensional brown adipose derived stem cell aggregates into brown adipose tissue in a second differentiation medium; and delivering the brown adipose tissue to the patient with a metabolic disorder.

Disclosed herein is a method of treating a patient with obesity. The method comprises: forming non-naturally occurring three-dimensional brown adipose derived stem cell aggregates; loading the non-naturally occurring three-dimensional brown adipose derived stem cell aggregates into an encapsulation system; differentiating the non-naturally occurring three-dimensional brown adipose derived stem cell aggregates into brown adipose tissue in a first differentiation medium; differentiating the non-naturally occurring three-dimensional brown adipose derived stem cell aggregates into brown adipose tissue in a second differentiation medium; and delivering the brown adipose tissue to the patient with obesity.

Disclosed herein is a method of treating a patient with an endocrine disorder. The method comprises: forming non-naturally occurring three-dimensional brown adipose derived stem cell aggregates; loading the non-naturally occurring three-dimensional brown adipose derived stem cell aggregates into an encapsulation system; differentiating the non-naturally occurring three-dimensional brown adipose derived stem cell aggregates into brown adipose tissue in a first differentiation medium; differentiating the non-naturally occurring three-dimensional brown adipose derived stem cell aggregates into brown adipose tissue in a second differentiation medium; and delivering the brown adipose tissue to the patient with an endocrine disorder.

Disclosed herein is a method of treating a patient with a cardiovascular disorder. The method comprises: forming non-naturally occurring three-dimensional brown adipose derived stem cell aggregates; loading the non-naturally occurring three-dimensional brown adipose derived stem cell aggregates into an encapsulation system; differentiating the non-naturally occurring three-dimensional brown adipose derived stem cell aggregates into brown adipose tissue in a first differentiation medium; differentiating the non-naturally occurring three-dimensional brown adipose derived stem cell aggregates into brown adipose tissue in a second differentiation medium; and delivering the brown adipose tissue to the patient with a cardiovascular disorder.

Disclosed herein is a method of treating a patient with liver disease. The method comprises: forming non-naturally occurring three-dimensional brown adipose derived stem cell aggregates; loading the non-naturally occurring three-dimensional brown adipose derived stem cell aggregates into an encapsulation system; differentiating the non-naturally occurring three-dimensional brown adipose derived stem cell aggregates into brown adipose tissue in a first differentiation medium; differentiating the non-naturally occurring three-dimensional brown adipose derived stem cell aggregates into brown adipose tissue in a second differentiation medium; and delivering the brown adipose tissue to the patient with liver disease.

### Definitions

In addition to the definitions previously set forth herein, the following definitions are relevant to the present disclosure:
The singular forms "a," "an," and "the" include plural references, unless the context clearly dictates otherwise.

A "2-dimensional (2D) culture" refers to cells spreading throughout the surface of a cell culture plate and adhering to the surface of the cell culture plate.

A "3-dimensional (3D) culture" refers to cells that do not adhere to the surface of a cell culture plate and instead associate with each other, thereby forming cellular aggregates.

Any numerical range recited in this specification describes all sub-ranges of the same numerical precision *(i.e.,* having the same number of specified digits) subsumed within the recited range. For example, a recited range of "1.0 to 10.0" describes all sub-ranges between (and including) the recited minimum value of 1.0 and the recited maximum value of 10.0, such as, for example, "2.4 to 7.6," even if the range of "2.4 to 7.6" is not expressly recited in the text of the specification. Accordingly, the Applicant reserves the right to amend this specification, including the claims, to expressly recite any sub-range of the same numerical precision subsumed within the ranges expressly recited in this specification. All such ranges are inherently described in this specification such that amending to expressly recite any such sub-ranges will comply with written description, sufficiency of description, and added matter requirements, including the requirements under 35 U.S.C. § 112(a) and Article 123(2) EPC. Also, unless expressly specified or otherwise required by context, all numerical parameters described in this specification (such as those expressing values, ranges, amounts, percentages, and the like) may be read as if prefaced by the word "about," even if the word "about" does not expressly appear before a number. Additionally, numerical parameters described in this specification should be construed in light of the number of reported significant digits, numerical precision, and by applying ordinary rounding techniques. It is also understood that numerical parameters described in this specification will necessarily possess the inherent variability characteristic of the underlying measurement techniques used to determine the numerical value of the parameter.

Any patent, publication, or other disclosure material identified herein is incorporated by reference into this specification in its entirety unless otherwise indicated, but only to the extent that the incorporated material does not conflict with existing descriptions, definitions, statements, or other disclosure material expressly set forth in this specification. As such, and to the extent necessary, the express disclosure as set forth in this specification supersedes any conflicting material incorporated by reference. Any material, or portion thereof, that is said to be incorporated by reference into this specification, but which conflicts with existing definitions, statements, or other disclosure material set forth herein, is only incorporated to the extent that no conflict arises between that incorporated material and the existing disclosure material. Applicants reserve the right to amend this specification to expressly recite any subject matter, or portion thereof, incorporated by reference herein.

The details of one or more aspects of the present disclosure are set forth in the accompanying examples below. Although any materials and methods similar or equivalent to those described herein can be used in the practice or testing of the present disclosure, specific examples of the materials and methods contemplated are now described. Other features, objects and advantages of the present disclosure will be apparent from the description. In the description examples, the singular forms also include the plural unless the context clearly dictates otherwise. Unless defined otherwise, all technical and scientific terms used herein have the same meaning as commonly understood by one of ordinary skill in the art to which this present disclosure belongs. In the case of conflict, the present description will control.

### Examples

The present disclosure will be more fully understood by reference to the following examples, which provide illustrative, non-limiting aspects of the invention.

### Example 1 - Differentiation of BADSC to brown adipocytes in differentiation medium comprising fetal bovine serum

BADSCs were isolated from fresh brown adipose tissue and were cultured for up to three passages. Cells were expanded in growth medium (GM) composed of Dulbecco's modified Eagle's medium (DMEM) low glucose (Gibco, Thermo Fisher Scientific, Waltham, Massachusetts, USA), supplemented with 10% human platelet lysate (Xcyte^{™} Plus Xeno-Free Supplement, iBiologics, Phoenix, Arizona, USA), 1% GlutaMAX^{™} Supplement (Gibco, Thermo Fisher Scientific), 1% Minimum Essential Medium Non-Essential Amino Acids (MEM-NEAA, Gibco, Thermo Fisher Scientific), 100 units/ml of penicillin and 100 µg/ml of streptomycin (Gibco, Thermo Fisher Scientific). Cells were seeded at a density of 3500 cells/cm² and medium was replaced every other day.

Adipocyte differentiation was induced two days after cells reached full confluency by addition of brown adipocyte differentiation medium 1 (AD-1). AD-1 is a serum based differentiation medium composed of DMEM low glucose (Gibco, Thermo Fisher Scientific) supplemented with 10% fetal bovine serum (FBS, HyClone, GE Healthcare, Life Sciences, Little Chalfont, Buckinghamshire, UK), 5 µM dexamethasone (MP Biomedicals, Santa Ana, California, USA), 500 µM 3-isobutyl-1-methylxanthine (IBMX, Sigma-Aldrich, St. Louis, Missouri, USA), 860 nM insulin (Gibco, Thermo Fisher Scientific), 125 nM indomethacin (Sigma-Aldrich), 1 nM triiodothyronine (T3, Sigma-Aldrich), 1 µM rosiglitazone (Sigma-Aldrich), 100 units/ml of penicillin, 100 µg/ml of streptomycin (Gibco, Thermo Fisher Scientific), and 2 mM L-glutamine (Gibco, Thermo Fisher Scientific).

### Example 2 - Differentiation of BADSC to brown adipocytes in a 2-step serum-free chemically defined differentiation medium

In order to develop a transplantable brown adipose tissue (BAT) for human applications, differentiation protocols applicable to cellular therapy in humans were sought.

BADSCs were isolated from fresh brown adipose tissue and were cultured for up to three passages. Cells were expanded in GM composed of DMEM low glucose (Gibco, Thermo Fisher Scientific), supplemented with 10% human platelet lysate (Xcyte^{™} Plus Xeno-Free Supplement, iBiologics), 1% GlutaMAX^{™} Supplement (Gibco, Thermo Fisher Scientific), 1% Minimum Essential Medium Non-Essential Amino Acids (MEM-NEAA, Gibco, Thermo Fisher Scientific), 100 units/ml of penicillin and 100 µg/ml of streptomycin (Gibco, Thermo Fisher Scientific). Cells were seeded at a density of 3500 cells/cm² and medium was replaced every other day.

Adipocyte differentiation was induced two days after cells reached full confluency by addition of brown adipocyte differentiation medium 2 (AD-2). AD-2 is a two-step xeno-free, serum free, chemically defined differentiation medium. In a first step, BADSC are grown in a first differentiation medium, AD-2 DIFF-1 culture medium, which comprises DMEM / Ham's F12 Media (1:1) (Lonza Group AG, Basel, Switzerland), 25 mM HEPES Buffer (Lonza Group AG), 2 mM L-glutamine (Gibco, Thermo Fisher Scientific), 1 µM dexamethasone (MP Biomedicals), 100 µM IBMX (Sigma-Aldrich), 860 nM insulin (Gibco, Thermo Fisher Scientific), 0.2 nM T3 (Sigma-Aldrich), 10 µg/ml apo-transferrin (Sigma-Aldrich), 100 units/ml of penicillin and 100 µg/ml of streptomycin (Gibco, Thermo Fisher Scientific). In a second step, after three days the AD-2 DIFF-1 culture medium was replaced with a second differentiation medium, AD-2 DIFF-2, a xeno-free, serum free, chemically defined differentiation medium, which comprises DMEM / Ham's F12 Media (1:1) (Lonza Group AG), 25 mM HEPES Buffer (Lonza Group AG), 2 mM L-glutamine (Gibco, Thermo Fisher Scientific), 860 nM insulin (Gibco, Thermo Fisher Scientific), 0.2 nM T3 (Sigma-Aldrich), 10 µg/ml apo-transferrin (Sigma-Aldrich), 100 units/ml of penicillin and 100 µg/ml of streptomycin (Gibco, Thermo Fisher Scientific) and 100 nM rosiglitazone.

In some examples, AD-2 can comprise human platelet lysate. In other examples, AD-2 does not comprise human platelet lysate.

The BADSC populations were differentiated in a xeno-free, serum free, chemically defined brown differentiation medium (AD-2 DIFF-1 and AD-2 DIFF-2) using a two-step method described above and its potency in generating brown adipocytes was compared to an FBS-based differentiation medium (AD-1) and to a commercially available adipogenic medium (StemPro^{™} Adipogenesis, Gibco, Thermo Fisher Scientific).

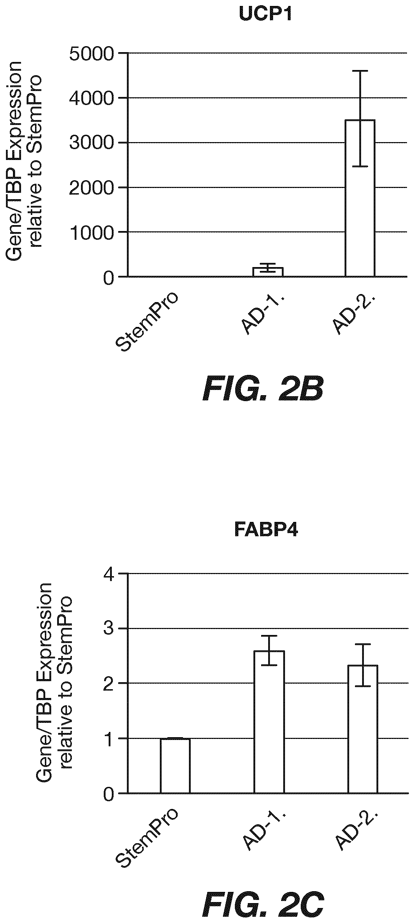

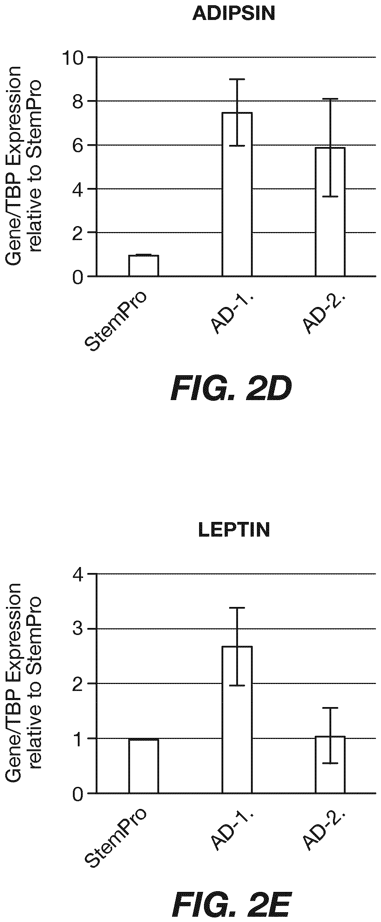

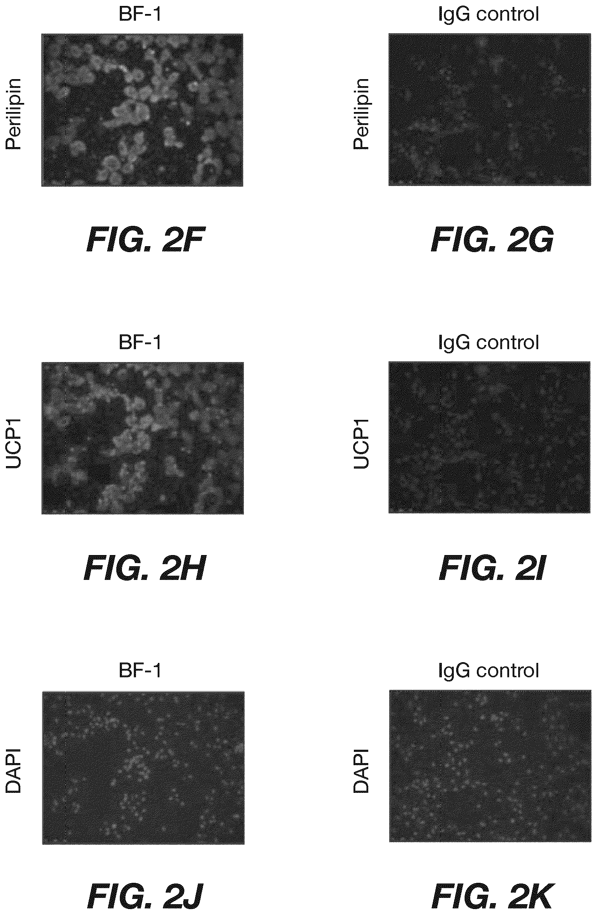

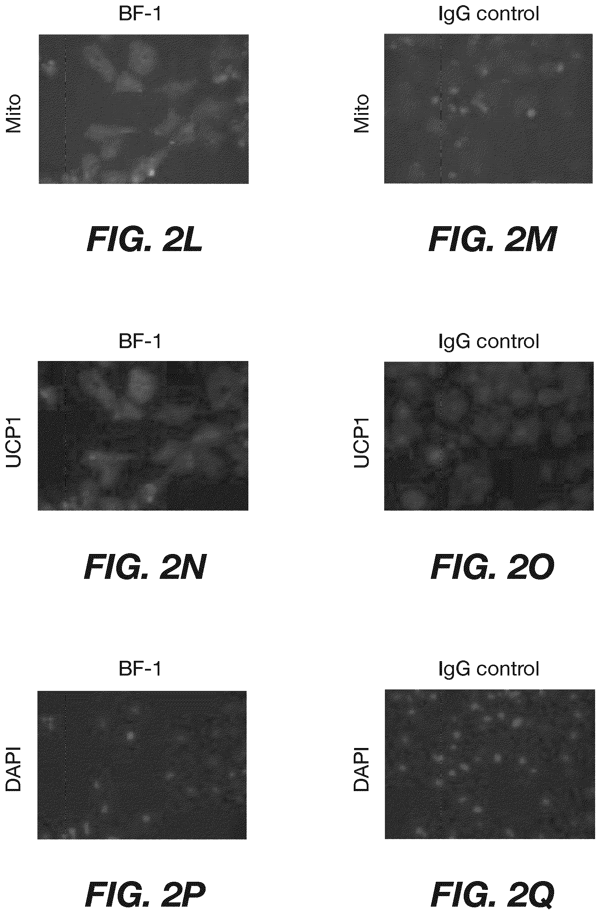

As demonstrated by the expression of the adipocyte markers FABP4 and adipsin (Figures 2C and 2D), AD-1 and AD-2 adipogenic media were comparably efficient at converting BADSC into adipocytes and more efficient than the commercial adipogenic medium, StemPro^{™} (Gibco, Thermo Fisher Scientific). Although AD-1 and AD-2 were equivalent in promoting adipocyte differentiation, adipocytes obtained in the xeno-free, serum free, chemically defined medium AD-2 were morphologically larger and contained larger lipid droplets (Figures 1A to 1M show cells cultured in AD-2; data not shown for cells cultured in AD-1). Differentiation using the AD-2 medium allowed for a much higher brown adipocyte differentiation than the AD-1 or the commercial adipogenic media.

Results also show that UCP1 gene expression was over 200 fold higher in AD-1 and over 3500 fold higher in AD-2 as compared to the commercial adipogenic medium, StemPro^{™} (Figure 2B). Additionally, the expression of the white specific marker leptin was 1.5 fold lower in AD-2 than AD-1 confirming the superior efficiency of AD-2 to direct BADSC to a brown adipose phenotype (Figure 2E).

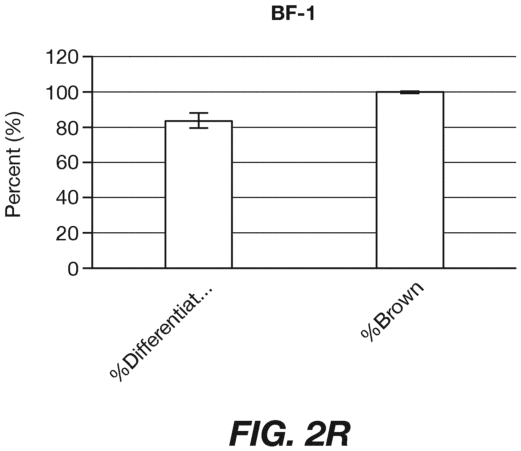

Immunocytochemistry analysis of BADSC population BF-1 differentiated in AD-2 for 15 days showed that the adipocyte conversion rate *i.e.* the percentage of cell positive for the adipocyte marker perilipin, is very high with over 80% of the cells differentiating into adipocytes (Figures 2F to 2K and Figure 2R). 98% of the differentiated cells (perilipin+ cells) co-expressed the brown specific marker UCP1 (Figures 2F to 2K and Figure 2R). This data confirmed the expression of UCP1 at the protein level (Figures 2H and 2I; Figures 2N and 20) and showed high yield of brown adipocyte conversion in the xeno-free, chemically defined differentiation medium. As expected, UCP1 protein localized in mitochondria as shown by the overlapping signals obtained when co-immunostaining differentiated BADSC for UCP1 and mitochondria (Figures 2N to 2Q).

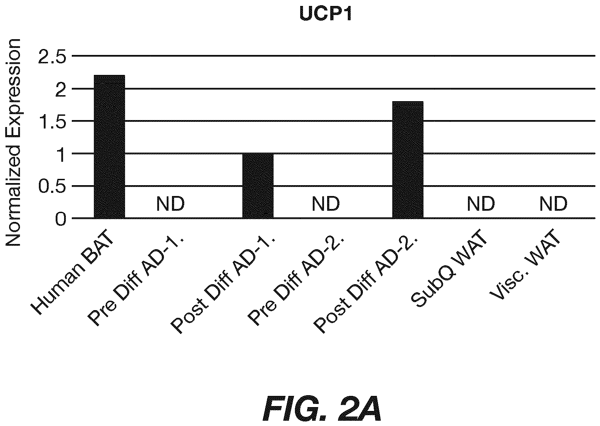

The results in Figures 2A to 2R demonstrate that that the 2-step AD-2 differentiation medium (AD-2 DIFF-1 and AD-2 DIFF-2) promotes a stronger brown adipocyte differentiation compared to AD-1 differentiation medium and the commercial adipogenic media.

### Example 3 - Method of making 3D BAGs

Non-naturally occurring 3-dimensional BADSC aggregates or BAGs were formed in non-adherent culture plates, such as AggreWellTM 400Ex 6-well plates (StemCell Technologies, Vancouver, British, Columbia, Canada).

BADSCs were first cultured in 2D using growth media under normoxia or hypoxia until 80% confluency. The non-adherent plates were coated with a rinsing solution, such as AggreWellTM rinsing solution (StemCell Technologies) following manufacturer's instructions. After washing the non-adherent plates with GM, 12 ml of cell suspension containing 2.4 million cells/ml in GM was loaded to each well of the non-adherent plates. The non-adherent plates were then centrifuged at 500g for 5 minutes using a swinging bucket centrifuge to allow the cells to settle uniformly into the microwells, resulting in a density of 1000 cells per microwell, thus creating uniform cellular aggregates. Without centrifugation, the non-naturally occurring 3-dimensional brown adipose-derived stem cell aggregates or BAGs will not be uniform.

The BAGs were then cultured in a non-adherent culture plate, such as AggreWellTM 400Ex 6-well plates, at 37°C in normoxia or hypoxia and 95% humidity for 24 hours in GM prior to harvest. Approximately 28200 BAGs were collected per non-adherent plate by gentle pipetting and resuspended in 800 µl of GM.

### Example 4 - A method of making three-dimensional brown adipose tissue in an encapsulation system

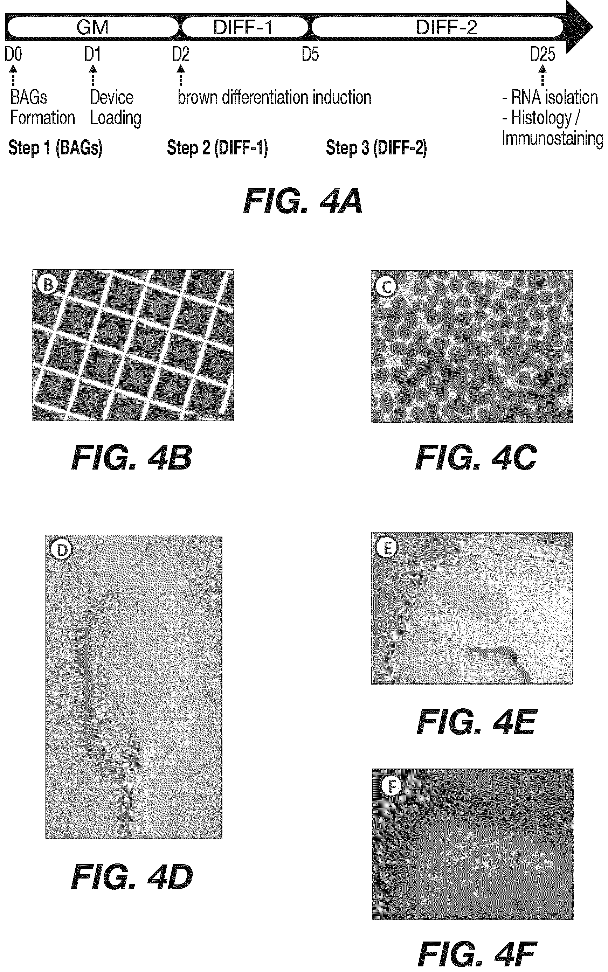

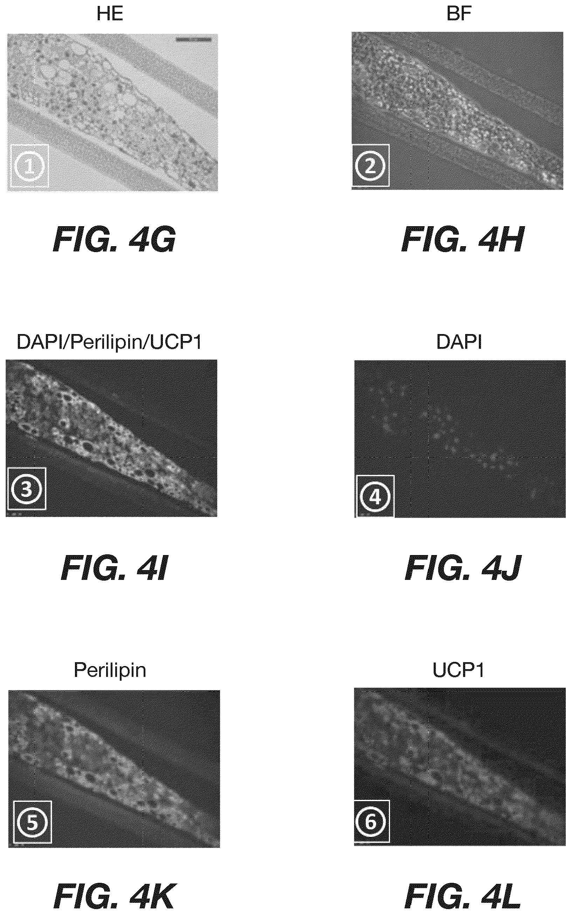

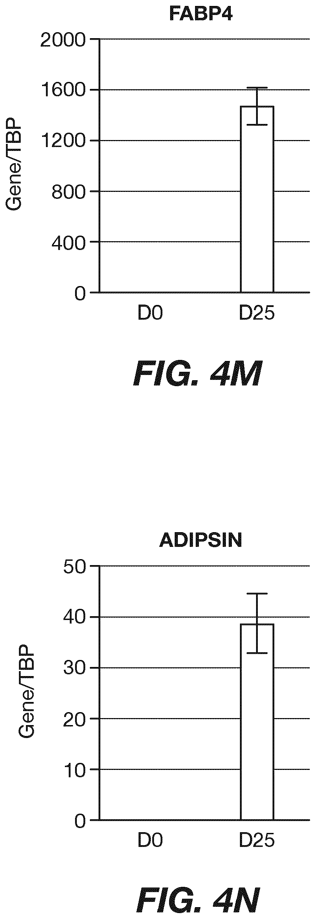

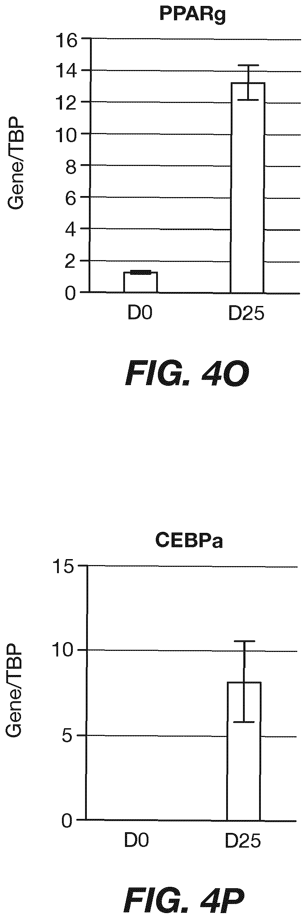

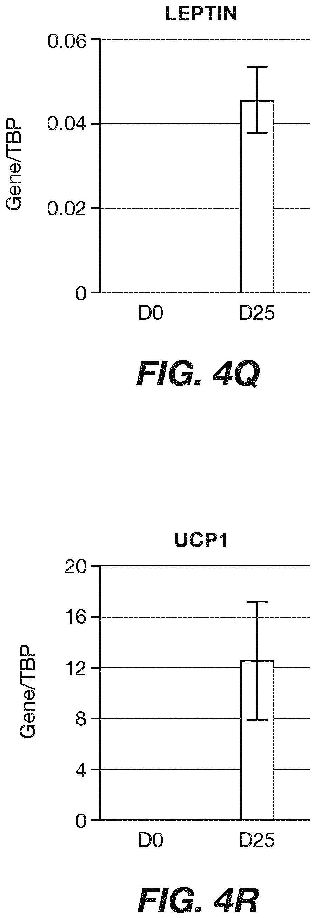

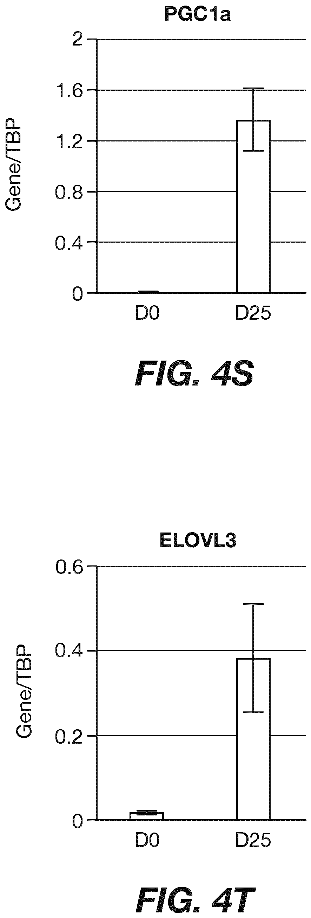

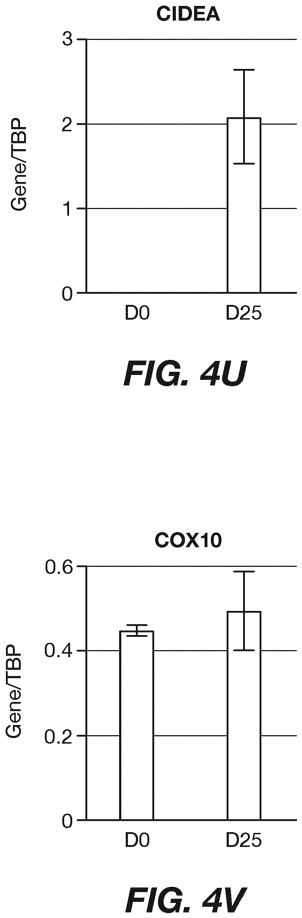

A differentiation protocol to efficiently differentiate BADSC into functional brown adipocytes in 3D culture within an encapsulation system, such as an encapsulation medical device, has been developed. This method, summarized in Figure 4A, consists of 3 steps: (1) forming non-naturally occurring three-dimensional BADSC aggregates (BAGs) in growth medium (≈160 µm/aggregate) (Figure 4B, 4C) and loading of BAGs into an encapsulation system, such as an encapsulation medical device (Figures 4D, 4E); (2) further differentiating the BAGS into brown adipose tissue (BAT) using the xeno-free, serum free, chemically defined AD-2-DIFF-1 medium; and (3) differentiating the BAGs into brown adipose tissue using the xeno-free, serum-free, chemically defined AD-2-DIFF-2 medium (Figure 4F).

In step 1: BAGs were formed in AggreWellTM 400Ex 6-well plates (StemCell Technologies) using BADSC population BF-1. The optimal cell plating density, in order to generate uniform BAGs, was determined to be 1000 cells per microwell. BAGs were subsequently loaded into the encapsulation system, such as an encapsulation medical device.
The BAGs suspension was loaded into an encapsulation device such as one Encaptra^{®} EN20 (ViaCyte) encapsulation device, using a Sureflo^{®} 20G catheter (Terumo Corporation, Tokyo, Japan). The device port was sealed with RTV Silicone Adhesive (NuSil Technology, Carpinteria, California, USA) and the encapsulated BAGs were cultured for 24 hours in 15 ml of GM in a 100 mm tissue culture dish. At that point, the BAGs merged and filled the entire volume of the encapsulation device. The resulting BAGs were highly uniform in size and shape, and uniform within and between experiments. Size can be easily modified by adjusting the cell seeding concentration formed in AggreWell^{™} 400Ex 6-well plates (StemCell Technologies). The optimal cell plating density in order to generate uniform BAGs was determined to be 1000 cells per microwell.

In step 2: the BAGs within the encapsulation medical device were differentiated for 3 days in vitro in a first differentiation medium called AD-2 DIFF-1 medium.

In step 3: the BAGs within the encapsulation medical device were further differentiated for 20 days in vitro in a second differentiation medium called AD-2 DIFF-2 medium.

Immunocytochemistry analysis showed that the non-naturally occurring brown adipose-derived stem cell aggregates comprising the BADSC population BF-1 efficiently differentiated into brown adipocytes in 3D inside the Encaptra^{®} encapsulation medical device. BADSC BF-1 cells differenting in the encapsulation medical device formed a tissue-like structure visualized by hematoxylin and eosin staining highly enriched in brown adipocytes (UCP1 positive and Perilipin positive) containing high contents of mitochondria (Figures 4G to 4L). These cells express high levels of adipocyte markers such as FABP4, adipsin, PPARg, CEBPa and leptin (Figures 4M to 4Q) and brown specific markers such as UCP1, PGC1a, CIDEA, ELOVL3, and COX10 (Figures 4R to 4V) as compared to undifferentiated BAGs.

In conclusion, it was shown that non-naturally occurring BADSC aggregates represent a very promising source of transplantable brown adipose tissue to increase energy expenditure and to potentially treat metabolic disorders, endocrine disorders, cardiovascular disorders, and liver diseases. Moreover, the strategy to use encapsulation to deliver the non-naturally occurring BADSC aggregates represents a safe delivery system and will help accelerate the development of BAT therapies for human applications.

### Example 5 - Evaluating efficacy and safety of BAGs delivered in Matrigel

Male SCID-beige mice (C.B-Igh-1b/GbmsTac-Prkdcscid-LystbgN7), 8 weeks old (Taconic Biosciences) were singly housed at 25°C and fed a high fat diet (HFT) comprising 60% fat (D12492, 60 kcal% fat [primarily lard], 20 kcal% carbohydrate). These mice have metabolic syndrome and cannot process glucose.

An encapsulation system was prepared by adding 1.6 x 10⁶ brown adipose derived stem cells (BADSCs) in 1 mL of 4 mg/mL matrigel (Corning^{®} Matrigel^{®} Matrix High Concentration (HC), Phenol-Red Free *LDEV-Free). The BADSCs were removed from their two-dimensional (2D) culture in cell adherent tissue culture flasks, added to non-adherent culture plates, and centrifuged. After centrifugation, the aggregates were uniform and added to the matrigel.

The encapsulation system (1 mL) was added to 20 wells of a 96-well plate (50 µL per well). After 1 hour of gelation, the encapsulation system became solid disks in the culture well. Growth media was added to the wells for 24 hours. The growth media was removed from the wells and AD-2 DIFF-1 was then added to the wells for 24 hours. The AD-2 DIFF-1 was removed from the wells and AD-2 DIFF-2 was then added to the wells for 14-21 days. After several days of culturing/differentiation, the encapsulation system comprising BAT forms a spherical shape *(i.e.* a bead). The beads contracted in size and reduced to 20-30 µl after the in vitro differentiation.

Forty beads were collected using a cell strainer. Forty beads comprise ~3.2 x 10⁶ total cells that make up the encapsulated BAT. The beads were transferred into a 1.5 mL conical vial, and placed on ice. 100 µl of cold 10 mg/mL matrigel was added to the beads, mixed well, and kept on ice.

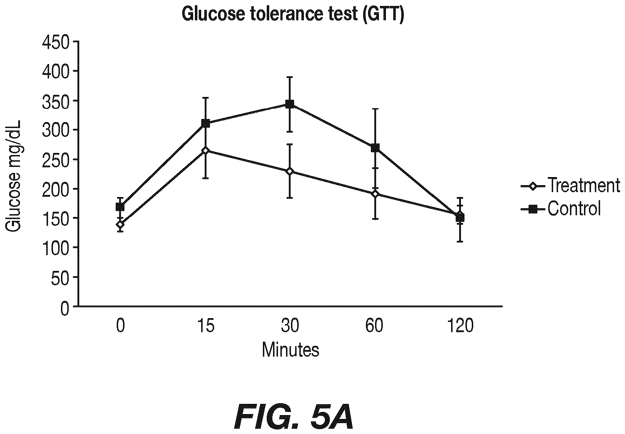

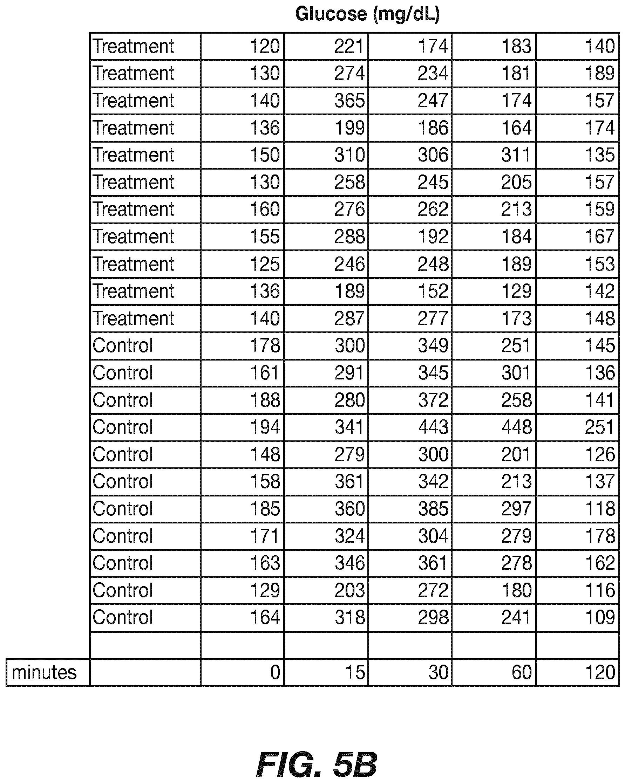

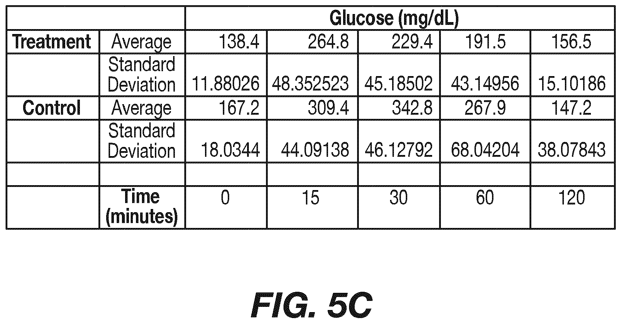

A small skin incision (~5 mm) was made near in the Interscapular brown fat pads of twenty-two (22) SCID-beige mice. If additional space was needed, then dorsal subQ sites were used. A spatula was used to lift the skin off the underneath white fat layer. The 40 beads in matrigel were delivered to the incision site in 11 of the 22 mice (Figures 5A-C, Treatment group) using a modified 1 mL micropipette tip and the incision was sutured. Matrigel alone was delivered to the incision site in the other 11 mice (Figure 5A-C, Control group) using a modified 1 mL micropipette tip and the incision was sutured.

Mice from the treatment group and mice from the control group were analyzed weekly to determine their ability to absorb glucose via a Glucose Tolerance Test (GTT). Prior to the analysis, these mice were fasted for 24 hours. After 24 hours, the mice were given an
intraperitoneal (IP) injection of glucose (1 mg/g of body weight) and the amount of glucose that was absorbed was measured using a blood sample at 0, 15, 30, 60, and 120 minutes post glucose injection.

Figure 5A-C show that mice transplanted with BAT (treatment group) were better able to absorb glucose over the course of 60 minutes at 4-weeks post-treatment (8-weeks post time induction of obesity) as compared to mice that were not transplanted with BAT (control group).

### Note Regarding Illustrative Examples

While the present disclosure provides descriptions of various specific aspects for the purpose of illustrating various examples of the present disclosure and/or its potential applications, it is understood that variations and modifications will occur to those skilled in the art. Accordingly, the invention or inventions described herein should be understood to be at least as broad as they are claimed, and not as more narrowly defined by particular illustrative examples provided herein.

## Claims

1. A method of making a non-naturally occurring three-dimensional brown adipose derived stem cell aggregate consisting of brown adipose derived stem cells that express one or more brown adipocyte genes in the absence of differentiation medium, the method comprising:
loading brown adipose derived stem cells grown in a two-dimensional (2D) culture into a non-adherent culture plate; and
centrifuging the non-adherent culture plate to uniformly position the brown adipose derived stem cells in the non-adherent culture plate, thereby forming non-naturally occurring three-dimensional brown adipose derived stem cell aggregates, wherein the aggregates consist of brown adipose derived stem cells.

2. The method of claim 1, further comprising:
prior to the loading, culturing the brown adipose derived stem cells in a two-dimensional (2D) culture using growth medium under normoxia or hypoxia.

3. The method of any one of the preceding claims, further comprising:
culturing the non-naturally occurring three-dimensional brown adipose derived stem cell aggregates in a non-adherent culture plate, such as an AggreWell^{™} 400Ex 6-well plate, at 37°C in normoxia or hypoxia and 95% humidity for 24 hours in a growth medium after centrifuging and prior to harvesting the non-naturally occurring three-dimensional brown adipose derived stem cell aggregates.

4. The method of any one of the preceding claims, further_comprising:
loading the non-naturally occurring three-dimensional brown adipose derived stem cell aggregates into an encapsulation system;
differentiating the non-naturally occurring three-dimensional brown adipose derived stem cell aggregates into brown adipose tissue in a first differentiation medium; and
further differentiating the non-naturally occurring three-dimensional brown adipose derived stem cell aggregates into brown adipose tissue in a second differentiation medium.

5. The method of claim 4, wherein the encapsulation system is selected from the group consisting of alginate microcapsules, cellulose hydrogels, red blood cells, porous polymer membranes, 3D biological scaffolds, polymers, PEG-based hydrogels, non-hydrogel beads, and matrigel.

6. The method of any one of claims 4 to 5, comprising
forming the non-naturally occurring three-dimensional brown adipose derived stem cell aggregates in a growth medium at ≈160 pm/aggregate before the loading of the non-naturally occurring three-dimensional brown adipose derived stem cell aggregates into the encapsulation system;
in the first differentiating step, using a xeno-free, serum free, chemically defined AD-2-DIFF-l medium as the first differentiation medium; and
in the second differentiating step, using a xeno- free, serum-free, chemically defined AD-2-DIFF-2 medium as the second differentiation medium.

7. The method of claim 6, comprising
in the first differentiating step, differentiating the aggregates within the encapsulation system for 3 days in-vitro in the first differentiation medium; and
in the second differentiating step, further differentiating the aggregates within the encapsulation medical device for 20 days in-vitro in the second differentiation medium.

8. The method of claim 4,
wherein the encapsulation system is an encapsulation medical device; or
wherein the first differentiation medium comprises dexamethasone, 3-isobutyl-1-methylxanthine (IBMX), and triiodothyronine (T3); or
wherein the second differentiation medium comprises T3 and rosiglitazone.

## Patentansprüche

1. Ein Verfahren zur Herstellung eines nichtnatürlich vorkommenden dreidimensionalen, aus braunem Fettgewebe gewonnenen Stammzellenaggregats, das aus aus braunem Fettgewebe gewonnenen Stammzellen besteht, die in Abwesenheit eines Differenzierungsmediums ein oder mehrere braune Adipozytengene exprimieren, wobei das Verfahren umfasst:
Laden von aus braunem Fettgewebe gewonnenen Stammzellen, die in einer zweidimensionalen (2D) Kultur gewachsen sind, in eine nicht-adhärente Kulturplatte; und
Zentrifugieren der nicht-adhärenten Kulturplatte, um die aus braunem Fettgewebe gewonnenen Stammzellen gleichmäßig in der nicht-adhärenten Kulturplatte zu positionieren, wodurch nichtnatürlich vorkommende dreidimensionale, aus braunem Fettgewebe gewonnene Stammzellenaggregate gebildet werden, wobei die Aggregate aus aus braunem Fettgewebe gewonnenen Stammzellen bestehen.

2. Das Verfahren nach Anspruch 1, zudem umfassend:
Kultivieren, vor dem Beladen, der aus braunem Fettgewebe gewonnenen Stammzellen in einer zweidimensionalen (2D) Kultur mit Wachstumsmedium unter Normoxie oder Hypoxie.

3. Das Verfahren nach einem der vorangegangenen Ansprüche, zudem umfassend:
Kultivieren der nichtnatürlich vorkommenden dreidimensionalen, aus braunem Fettgewebe gewonnenen Stammzellenaggregate in einer nicht-adhärenten Kulturplatte, wie beispielsweise einer AggreWell^{™} 400Ex 6-Well-Platte, bei 37°C in Normoxie oder Hypoxie und 95% Luftfeuchtigkeit für 24 Stunden in einem Wachstumsmedium nach dem Zentrifugieren und vor dem Ernten der nichtnatürlich vorkommenden dreidimensionalen, aus braunem Fettgewebe gewonnenen Stammzellenaggregate.

4. Das Verfahren nach einem der vorangegangenen Ansprüche, zudem umfassend:
Laden der nichtnatürlich vorkommenden dreidimensionalen, aus braunem Fettgewebe gewonnenen Stammzellenaggregate in ein Verkapselungssystem;
Differenzieren der nichtnatürlich vorkommenden dreidimensionalen, aus braunem Fettgewebe gewonnenen Stammzellenaggregate zu Gewebe aus braunem Fettgewebe in einem ersten Differenzierungsmedium; und
weiteres Differenzieren der nichtnatürlich vorkommenden dreidimensionalen, aus braunem Fettgewebe gewonnenen Stammzellenaggregate in einem zweiten Differenzierungsmedium zu Gewebe aus braunem Fettgewebe.

5. Das Verfahren nach Anspruch 4, wobei das Verkapselungssystem aus der Gruppe ausgewählt ist, die aus Alginat-Mikrokapseln, Cellulose-Hydrogelen, roten Blutkörperchen, porösen Polymermembranen, biologischen 3D-Gerüsten, Polymeren, Hydrogelen auf PEG-Basis, Nicht-Hydrogel-Perlen und Matrigel besteht.

6. Das Verfahren nach einem der Ansprüche 4 bis 5, umfassend
Bilden der nichtnatürlich vorkommenden dreidimensionalen, aus braunem Fettgewebe gewonnenen Stammzellenaggregate in einem Wachstumsmedium bei ≈160 pm/Aggregat vor dem Laden der nichtnatürlich vorkommenden dreidimensionalen, aus braunem Fettgewebe gewonnenen Stammzellenaggregate in das Verkapselungssystem;
im ersten Differenzierungsschritt Verwenden eines xenofreien, serumfreien, chemisch definierten AD-2-DIFF-1-Mediums als das erste Differenzierungsmedium; und
im zweiten Differenzierungsschritt Verwenden eines xenofreien, serumfreien, chemisch definierten AD-2-DIFF-2-Mediums als das zweite Differenzierungsmedium.

7. Das Verfahren nach Anspruch 6, umfassend
im ersten Differenzierungsschritt Differenzieren der Aggregate innerhalb des Verkapselungssystems für 3 Tage in vitro in dem ersten Differenzierungsmedium; und
im zweiten Differenzierungsschritt weiteres Differenzieren der Aggregate innerhalb der medizinischen Verkapselungsvorrichtung für 20 Tage in vitro in dem zweiten Differenzierungsmedium.

8. Das Verfahren nach Anspruch 4,
wobei das Verkapselungssystem eine medizinische Verkapselungsvorrichtung ist; oder
wobei das erste Differenzierungsmedium Dexamethason, 3-Isobutyl-1-methylxanthin (IBMX) und Trijodthyronin (T3) enthält; oder
wobei das zweite Differenzierungsmedium T3 und Rosiglitazon enthält.

## Revendications

1. Un procédé de production d'un agrégat de cellules souches dérivées de l'adipeuse brune tridimensionnel d'origine non naturel qui expriment un ou plusieurs gènes de l'adipocyte brun en l'absence de milieu de différenciation, le procédé comprenant :
charger des cellules souches dérivées de l'adipeuse brune, qui ont poussée dans une culture bidimensionnelle (2D), dans une plaque de culture non adhérente ; et
centrifuger la plaque de culture non adhérente pour positionner uniformément les cellules souches dérivées de l'adipeuse brune dans la plaque de culture non adhérente, formant ainsi des agrégats de cellules souches dérivées de l'adipeuse brune tridimensionnels d'origine non naturel, les agrégats étant constitués de cellules souches dérivées de l'adipeuse brune.

2. Le procédé selon la revendication 1, comprenant en outre :
avant le chargement, culturer les cellules souches dérivées de l'adipeuse brune dans une culture bidimensionnelle (2D) à l'aide d'un milieu de croissance en normoxie ou en hypoxie.

3. Le procédé selon l'une des revendications précédentes, comprenant en outre :
cultiver les agrégats de cellules souches dérivées de l'adipeuse brune tridimensionnels d'origine non naturel dans une plaque de culture non adhérente, telle qu'une plaque AggreWell^{™} 400Ex à 6 puits, à 37°C en normoxie ou en hypoxie et à 95 % d'humidité pendant 24 heures dans un milieu de croissance après la centrifugation et avant la récolte des agrégats de cellules souches dérivées de l'adipeuse brune tridimensionnels d'origine non naturel.

4. Le procédé selon l'une des revendications précédentes, comprenant en outre :
charger des agrégats de cellules souches dérivées de l'adipeuse brune tridimensionnels d'origine non naturel, dans un système d'encapsulation ;
différencier les agrégats de cellules souches dérivées de l'adipeuse brune tridimensionnels d'origine non naturel en tissu adipeux brun dans un premier milieu de différenciation ; et
différencier en plus les agrégats de cellules souches dérivées de l'adipeuse brune tridimensionnels d'origine non naturel en tissu adipeux brun dans un deuxième milieu de différenciation.

5. Le procédé selon la revendication 4, dans lequel le système d'encapsulation est choisi dans le groupe constitué par les microcapsules d'alginate, les hydrogels de cellulose, les globules rouges, les membranes polymères poreuses, les échafaudages biologiques 3D, les polymères, les hydrogels à base de PEG, les billes non hydrogel et le matrigel.

6. Le procédé selon l'une des revendications 4 à 5 de, comprenant
former les agrégats de cellules souches dérivées de l'adipeuse brune tridimensionnels d'origine non naturel dans un milieu de croissance à ≈160 pm/agrégat avant le chargement des agrégats de cellules souches dérivées de l'adipeuse brune tridimensionnels d'origine non naturel dans le système d'encapsulation ;
dans la première étape de différenciation, utiliser un milieu AD-2-DIFF-l sans xéno, sans sérum et chimiquement défini comme le premier milieu de différenciation ; et
dans la deuxième étape de différenciation, utiliser un milieu AD-2-DIFF-2 sans xéno, sans sérum et chimiquement défini comme le deuxième milieu de différenciation.

7. Le procédé selon la revendication 6, comprenant
dans la première étape de différenciation, différencier les agrégats à l'intérieur du système d'encapsulation pendant 3 jours in vitro dans le premier milieu de différenciation ; et
dans la deuxième étape de différenciation, différencier en plus les agrégats à l'intérieur du dispositif médical d'encapsulation pendant 20 jours in vitro dans le deuxième milieu de différenciation.

8. Le procédé selon la revendication 4,
dans lequel le système d'encapsulation est un dispositif médical d'encapsulation ; ou
dans lequel le premier milieu de différenciation comprend de la dexaméthasone, de la 3-isobutyl-1-méthylxanthine (IBMX) et de la triiodothyronine (T3) ; ou
dans lequel le deuxième milieu de différenciation comprend de la T3 et de la rosiglitazone.
